(19) Europäisches Patentamt
European Patent Office
Office européen des brevets

(11) **EP 3 550 305 A1**

(12) **EUROPEAN PATENT APPLICATION**
published in accordance with Art. 153(4) EPC

(43) Date of publication:
**09.10.2019 Bulletin 2019/41**

(51) Int Cl.:
**G01N 33/68** (2006.01)

(21) Application number: **17875457.8**

(22) Date of filing: **30.11.2017**

(86) International application number:
**PCT/JP2017/043133**

(87) International publication number:
**WO 2018/101427 (07.06.2018 Gazette 2018/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **01.12.2016 US 201662428823 P**

(71) Applicant: **Santen Pharmaceutical Co., Ltd.**
**Higashiyodogawa-ku**
**Osaka-shi**
**Osaka 533-8651 (JP)**

(72) Inventors:
• **SHIBAGAKI, Keiichi**
**Ikoma-shi**
**Nara 630-0101 (JP)**

• **TOSHIMORI, Masanao**
**Osaka-shi**
**Osaka 530-8552 (JP)**
• **OKA, Koji**
**Emeryville, California 94608 (US)**
• **MAEDA, Shingo**
**Osaka-shi**
**Osaka 530-8552 (JP)**
• **INOUE, Hiroshi**
**Osaka-shi**
**Osaka 530-8552 (JP)**

(74) Representative: **Müller-Boré & Partner**
**Patentanwälte PartG mbB**
**Friedenheimer Brücke 21**
**80639 München (DE)**

(54) **METHOD FOR PREDICTING EFFICACY OF ANTI-VEGF DRUG TREATMENT FOR EXUDATIVE AGE-RELATED MACULAR DEGENERATION**

(57) The disclosure provides a method of predicting the responsiveness of a wet AMD patient to anti-VEGF therapy comprising (1) determining the level of at least one marker protein selected from the group consisting of TGF-beta, BMP9, angiopoietin-1, and angiopoietin-2 in a blood, plasma or serum sample obtained from the patient, and (2) predicting the responsiveness of the patient to the anti-VEGF therapy with reference to the level determined in step (1), as well as a diagnostic agent for use in the method.

Fig. 1

SEQUENCE LISTING

<110>  Santen Pharmaceutical Co., Ltd.

<120>  Method of predicting effectiveness of treatment of exudative
       age-related macular degeneration with anti-VEGF agent in patient

<130>  673788

<150>  US 62/428823
<151>  2016-12-01

<160>  4

<170>  PatentIn version 3.5

<210>  1
<211>  390
<212>  PRT
<213>  Homo sapiens

<400>  1

Met Pro Pro Ser Gly Leu Arg Leu Leu Leu Leu Leu Leu Pro Leu Leu
1               5                   10                  15


Trp Leu Leu Val Leu Thr Pro Gly Arg Pro Ala Ala Gly Leu Ser Thr
            20                  25                  30


Cys Lys Thr Ile Asp Met Glu Leu Val Lys Arg Lys Arg Ile Glu Ala
            35                  40                  45


Ile Arg Gly Gln Ile Leu Ser Lys Leu Arg Leu Ala Ser Pro Pro Ser
        50                  55                  60


Gln Gly Glu Val Pro Pro Gly Pro Leu Pro Glu Ala Val Leu Ala Leu
65                  70                  75                  80


Tyr Asn Ser Thr Arg Asp Arg Val Ala Gly Glu Ser Ala Glu Pro Glu
                85                  90                  95


Pro Glu Pro Glu Ala Asp Tyr Tyr Ala Lys Glu Val Thr Arg Val Leu
            100                 105                 110


Met Val Glu Thr His Asn Glu Ile Tyr Asp Lys Phe Lys Gln Ser Thr
            115                 120                 125


His Ser Ile Tyr Met Phe Phe Asn Thr Ser Glu Leu Arg Glu Ala Val
        130                 135                 140


Pro Glu Pro Val Leu Leu Ser Arg Ala Glu Leu Arg Leu Leu Arg Leu
145                 150                 155                 160

```
Lys Leu Lys Val Glu Gln His Val Glu Leu Tyr Gln Lys Tyr Ser Asn
                165             170             175

Asn Ser Trp Arg Tyr Leu Ser Asn Arg Leu Leu Ala Pro Ser Asp Ser
            180             185             190

Pro Glu Trp Leu Ser Phe Asp Val Thr Gly Val Val Arg Gln Trp Leu
        195             200             205

Ser Arg Gly Gly Glu Ile Glu Gly Phe Arg Leu Ser Ala His Cys Ser
    210             215             220

Cys Asp Ser Arg Asp Asn Thr Leu Gln Val Asp Ile Asn Gly Phe Thr
225             230             235             240

Thr Gly Arg Arg Gly Asp Leu Ala Thr Ile His Gly Met Asn Arg Pro
            245             250             255

Phe Leu Leu Leu Met Ala Thr Pro Leu Glu Arg Ala Gln His Leu Gln
        260             265             270

Ser Ser Arg His Arg Arg Ala Leu Asp Thr Asn Tyr Cys Phe Ser Ser
        275             280             285

Thr Glu Lys Asn Cys Cys Val Arg Gln Leu Tyr Ile Asp Phe Arg Lys
    290             295             300

Asp Leu Gly Trp Lys Trp Ile His Glu Pro Lys Gly Tyr His Ala Asn
305             310             315             320

Phe Cys Leu Gly Pro Cys Pro Tyr Ile Trp Ser Leu Asp Thr Gln Tyr
            325             330             335

Ser Lys Val Leu Ala Leu Tyr Asn Gln His Asn Pro Gly Ala Ser Ala
            340             345             350

Ala Pro Cys Cys Val Pro Gln Ala Leu Glu Pro Leu Pro Ile Val Tyr
        355             360             365

Tyr Val Gly Arg Lys Pro Lys Val Glu Gln Leu Ser Asn Met Ile Val
    370             375             380

Arg Ser Cys Lys Cys Ser
385             390


<210>   2
<211>   429
<212>   PRT
```

<213> Homo sapiens

<400> 2

Met Cys Pro Gly Ala Leu Trp Val Ala Leu Pro Leu Leu Ser Leu Leu
1               5                   10                  15

Ala Gly Ser Leu Gln Gly Lys Pro Leu Gln Ser Trp Gly Arg Gly Ser
            20                  25                  30

Ala Gly Gly Asn Ala His Ser Pro Leu Gly Val Pro Gly Gly Gly Leu
            35                  40                  45

Pro Glu His Thr Phe Asn Leu Lys Met Phe Leu Glu Asn Val Lys Val
        50                  55                  60

Asp Phe Leu Arg Ser Leu Asn Leu Ser Gly Val Pro Ser Gln Asp Lys
65                  70                  75                  80

Thr Arg Val Glu Pro Pro Gln Tyr Met Ile Asp Leu Tyr Asn Arg Tyr
                85                  90                  95

Thr Ser Asp Lys Ser Thr Thr Pro Ala Ser Asn Ile Val Arg Ser Phe
                100                 105                 110

Ser Met Glu Asp Ala Ile Ser Ile Thr Ala Thr Glu Asp Phe Pro Phe
            115                 120                 125

Gln Lys His Ile Leu Leu Phe Asn Ile Ser Ile Pro Arg His Glu Gln
        130                 135                 140

Ile Thr Arg Ala Glu Leu Arg Leu Tyr Val Ser Cys Gln Asn His Val
145                 150                 155                 160

Asp Pro Ser His Asp Leu Lys Gly Ser Val Val Ile Tyr Asp Val Leu
                165                 170                 175

Asp Gly Thr Asp Ala Trp Asp Ser Ala Thr Glu Thr Lys Thr Phe Leu
            180                 185                 190

Val Ser Gln Asp Ile Gln Asp Glu Gly Trp Glu Thr Leu Glu Val Ser
            195                 200                 205

Ser Ala Val Lys Arg Trp Val Arg Ser Asp Ser Thr Lys Ser Lys Asn
        210                 215                 220

Lys Leu Glu Val Thr Val Glu Ser His Arg Lys Gly Cys Asp Thr Leu
225                 230                 235                 240

```
Asp Ile Ser Val Pro Pro Gly Ser Arg Asn Leu Pro Phe Phe Val Val
            245             250             255

Phe Ser Asn Asp His Ser Ser Gly Thr Lys Glu Thr Arg Leu Glu Leu
            260             265             270

Arg Glu Met Ile Ser His Glu Gln Glu Ser Val Leu Lys Lys Leu Ser
            275             280             285

Lys Asp Gly Ser Thr Glu Ala Gly Glu Ser Ser His Glu Glu Asp Thr
            290             295             300

Asp Gly His Val Ala Ala Gly Ser Thr Leu Ala Arg Arg Lys Arg Ser
305             310             315             320

Ala Gly Ala Gly Ser His Cys Gln Lys Thr Ser Leu Arg Val Asn Phe
                325             330             335

Glu Asp Ile Gly Trp Asp Ser Trp Ile Ile Ala Pro Lys Glu Tyr Glu
                340             345             350

Ala Tyr Glu Cys Lys Gly Gly Cys Phe Phe Pro Leu Ala Asp Asp Val
            355             360             365

Thr Pro Thr Lys His Ala Ile Val Gln Thr Leu Val His Leu Lys Phe
    370             375             380

Pro Thr Lys Val Gly Lys Ala Cys Cys Val Pro Thr Lys Leu Ser Pro
385             390             395             400

Ile Ser Val Leu Tyr Lys Asp Asp Met Gly Val Pro Thr Leu Lys Tyr
            405             410             415

His Tyr Glu Gly Met Ser Val Ala Glu Cys Gly Cys Arg
            420             425
```

```
<210>   3
<211>   498
<212>   PRT
<213>   Homo sapiens

<400>   3

Met Thr Val Phe Leu Ser Phe Ala Phe Leu Ala Ala Ile Leu Thr His
1               5               10              15

Ile Gly Cys Ser Asn Gln Arg Arg Ser Pro Glu Asn Ser Gly Arg Arg
            20              25              30
```

Tyr Asn Arg Ile Gln His Gly Gln Cys Ala Tyr Thr Phe Ile Leu Pro
        35                  40                  45

Glu His Asp Gly Asn Cys Arg Glu Ser Thr Thr Asp Gln Tyr Asn Thr
        50                  55                  60

Asn Ala Leu Gln Arg Asp Ala Pro His Val Glu Pro Asp Phe Ser Ser
65                  70                  75                  80

Gln Lys Leu Gln His Leu Glu His Val Met Glu Asn Tyr Thr Gln Trp
                85                  90                  95

Leu Gln Lys Leu Glu Asn Tyr Ile Val Glu Asn Met Lys Ser Glu Met
            100                 105                 110

Ala Gln Ile Gln Gln Asn Ala Val Gln Asn His Thr Ala Thr Met Leu
            115                 120                 125

Glu Ile Gly Thr Ser Leu Leu Ser Gln Thr Ala Glu Gln Thr Arg Lys
    130                 135                 140

Leu Thr Asp Val Glu Thr Gln Val Leu Asn Gln Thr Ser Arg Leu Glu
145                 150                 155                 160

Ile Gln Leu Leu Glu Asn Ser Leu Ser Thr Tyr Lys Leu Glu Lys Gln
                165                 170                 175

Leu Leu Gln Gln Thr Asn Glu Ile Leu Lys Ile His Glu Lys Asn Ser
            180                 185                 190

Leu Leu Glu His Lys Ile Leu Glu Met Glu Gly Lys His Lys Glu Glu
            195                 200                 205

Leu Asp Thr Leu Lys Glu Glu Lys Glu Asn Leu Gln Gly Leu Val Thr
    210                 215                 220

Arg Gln Thr Tyr Ile Ile Gln Glu Leu Glu Lys Gln Leu Asn Arg Ala
225                 230                 235                 240

Thr Thr Asn Asn Ser Val Leu Gln Lys Gln Gln Leu Glu Leu Met Asp
                245                 250                 255

Thr Val His Asn Leu Val Asn Leu Cys Thr Lys Glu Gly Val Leu Leu
            260                 265                 270

Lys Gly Gly Lys Arg Glu Glu Glu Lys Pro Phe Arg Asp Cys Ala Asp
    275                 280                 285

```
Val Tyr Gln Ala Gly Phe Asn Lys Ser Gly Ile Tyr Thr Ile Tyr Ile
    290             295             300

Asn Asn Met Pro Glu Pro Lys Lys Val Phe Cys Asn Met Asp Val Asn
305             310             315             320

Gly Gly Gly Trp Thr Val Ile Gln His Arg Glu Asp Gly Ser Leu Asp
            325             330             335

Phe Gln Arg Gly Trp Lys Glu Tyr Lys Met Gly Phe Gly Asn Pro Ser
        340             345             350

Gly Glu Tyr Trp Leu Gly Asn Glu Phe Ile Phe Ala Ile Thr Ser Gln
        355             360             365

Arg Gln Tyr Met Leu Arg Ile Glu Leu Met Asp Trp Glu Gly Asn Arg
    370             375             380

Ala Tyr Ser Gln Tyr Asp Arg Phe His Ile Gly Asn Glu Lys Gln Asn
385             390             395             400

Tyr Arg Leu Tyr Leu Lys Gly His Thr Gly Thr Ala Gly Lys Gln Ser
            405             410             415

Ser Leu Ile Leu His Gly Ala Asp Phe Ser Thr Lys Asp Ala Asp Asn
            420             425             430

Asp Asn Cys Met Cys Lys Cys Ala Leu Met Leu Thr Gly Gly Trp Trp
            435             440             445

Phe Asp Ala Cys Gly Pro Ser Asn Leu Asn Gly Met Phe Tyr Thr Ala
        450             455             460

Gly Gln Asn His Gly Lys Leu Asn Gly Ile Lys Trp His Tyr Phe Lys
465             470             475             480

Gly Pro Ser Tyr Ser Leu Arg Ser Thr Thr Met Met Ile Arg Pro Leu
            485             490             495

Asp Phe
```

```
<210>   4
<211>   496
<212>   PRT
<213>   Homo sapiens
```

<400> 4

Met Trp Gln Ile Val Phe Phe Thr Leu Ser Cys Asp Leu Val Leu Ala
1               5                   10                  15

Ala Ala Tyr Asn Asn Phe Arg Lys Ser Met Asp Ser Ile Gly Lys Lys
            20                  25                  30

Gln Tyr Gln Val Gln His Gly Ser Cys Ser Tyr Thr Phe Leu Leu Pro
        35                  40                  45

Glu Met Asp Asn Cys Arg Ser Ser Ser Ser Pro Tyr Val Ser Asn Ala
    50                  55                  60

Val Gln Arg Asp Ala Pro Leu Glu Tyr Asp Asp Ser Val Gln Arg Leu
65                  70                  75                  80

Gln Val Leu Glu Asn Ile Met Glu Asn Asn Thr Gln Trp Leu Met Lys
                85                  90                  95

Leu Glu Asn Tyr Ile Gln Asp Asn Met Lys Lys Glu Met Val Glu Ile
            100                 105                 110

Gln Gln Asn Ala Val Gln Asn Gln Thr Ala Val Met Ile Glu Ile Gly
        115                 120                 125

Thr Asn Leu Leu Asn Gln Thr Ala Glu Gln Thr Arg Lys Leu Thr Asp
    130                 135                 140

Val Glu Ala Gln Val Leu Asn Gln Thr Thr Arg Leu Glu Leu Gln Leu
145                 150                 155                 160

Leu Glu His Ser Leu Ser Thr Asn Lys Leu Glu Lys Gln Ile Leu Asp
                165                 170                 175

Gln Thr Ser Glu Ile Asn Lys Leu Gln Asp Lys Asn Ser Phe Leu Glu
        180                 185                 190

Lys Lys Val Leu Ala Met Glu Asp Lys His Ile Ile Gln Leu Gln Ser
        195                 200                 205

Ile Lys Glu Glu Lys Asp Gln Leu Gln Val Leu Val Ser Lys Gln Asn
    210                 215                 220

Ser Ile Ile Glu Glu Leu Glu Lys Lys Ile Val Thr Ala Thr Val Asn
225                 230                 235                 240

Asn Ser Val Leu Gln Lys Gln Gln His Asp Leu Met Glu Thr Val Asn

245           250           255

Asn Leu Leu Thr Met Met Ser Thr Ser Asn Ser Ala Lys Asp Pro Thr
        260           265        270

Val Ala Lys Glu Glu Gln Ile Ser Phe Arg Asp Cys Ala Glu Val Phe
      275         280        285

Lys Ser Gly His Thr Thr Asn Gly Ile Tyr Thr Leu Thr Phe Pro Asn
    290         295        300

Ser Thr Glu Glu Ile Lys Ala Tyr Cys Asp Met Glu Ala Gly Gly Gly
305          310        315        320

Gly Trp Thr Ile Ile Gln Arg Arg Glu Asp Gly Ser Val Asp Phe Gln
        325        330        335

Arg Thr Trp Lys Glu Tyr Lys Val Gly Phe Gly Asn Pro Ser Gly Glu
        340        345        350

Tyr Trp Leu Gly Asn Glu Phe Val Ser Gln Leu Thr Asn Gln Gln Arg
        355        360        365

Tyr Val Leu Lys Ile His Leu Lys Asp Trp Glu Gly Asn Glu Ala Tyr
    370         375        380

Ser Leu Tyr Glu His Phe Tyr Leu Ser Ser Glu Glu Leu Asn Tyr Arg
385          390        395        400

Ile His Leu Lys Gly Leu Thr Gly Thr Ala Gly Lys Ile Ser Ser Ile
        405        410        415

Ser Gln Pro Gly Asn Asp Phe Ser Thr Lys Asp Gly Asp Asn Asp Lys
        420        425        430

Cys Ile Cys Lys Cys Ser Gln Met Leu Thr Gly Gly Trp Trp Phe Asp
        435        440        445

Ala Cys Gly Pro Ser Asn Leu Asn Gly Met Tyr Tyr Pro Gln Arg Gln
        450        455        460

Asn Thr Asn Lys Phe Asn Gly Ile Lys Trp Tyr Tyr Trp Lys Gly Ser
465          470        475        480

Gly Tyr Ser Leu Lys Ala Thr Thr Met Met Ile Arg Pro Ala Asp Phe
        485        490        495

**Description**

TECHNICAL FIELD

This application claims the benefit of priority of the prior US Provisional Application (US Provisional Application No. 62/428823), the entire contents of which are incorporated herein by reference.

The disclosure relates to a method of predicting the responsiveness of a wet AMD patient to anti-VEGF therapy or a diagnostic agent for use in the method.

BACKGROUND ART

One main cause of social blindness in the elderlies is age-related macular degeneration (AMD). Two types of AMD are known: atrophic (dry) AMD, which is associated with geographic atrophy (i.e., atrophy of retinal pigment epithelium and choroidal microvasculature), and exudative (wet) AMD, which is characterized by choroidal neovascularization (CNV) under retina or retinal pigment epithelium. Involvement of vascular endothelial growth factors (VEGFs) in the CNV formation, the main pathology of wet AMD, has been revealed and several anti-VEGF agents have been approved for treating wet AMD.

The anti-VEGF agents are known to exhibit good clinical effects. The agents, however, are expensive and cause large mental and physical burdens on patients because they are administered by intravitreal injection. Furthermore, some patients are refractory to the anti-VEGF therapy. For example, Non-Patent Literature 1 discloses that improvement in visual acuity was displayed by 40 % of patients, deterioration by 25 % of patients and no change by 35 % of patients after treatment with an anti-VEGF agent. No method is available for predicting whether a patient is refractory to the anti-VEGF therapy.

Some anti-VEGF agents are also used for treating a cancer. When the cancer is refractory to the anti-VEGF therapy, use of inhibitors for other molecules modulating angiogenesis might be suggested (Non-Patent Literature 2 and 3). However, the role of the molecules modulating angiogenesis in the development of the refractoriness to the anti-VEGF therapy has not been revealed.

REFERENCES

NON-PATENT LITERATURE

[Non-Patent Literature 1] Graefes Arch Clin Exp Ophthalmol. 2014;252(4):647-55.
[Non-Patent Literature 2] Curr Oncol Rep. 2014;16(2):365.
[Non-Patent Literature 3] J Clin Oncol 2010;28(15):suppl; abstract 4630.

SUMMARY OF THE INVENTION

One of the objects of the disclosure is to provide a method of predicting the responsiveness of a wet AMD patient to the anti-VEGF therapy, or a diagnostic agent for use in the method.

The inventors found that the serum levels of TGF-beta, BMP9, angiopoietin-1, and angiopoietin-2 in wet AMD patients correlate with the responsiveness of the patients to the anti-VEGF therapy.

Accordingly, in an aspect the disclosure provides a method of predicting the responsiveness of a wet AMD patient to anti-VEGF therapy comprising

(1) determining the level of at least one marker protein selected from the group consisting of TGF-beta, BMP9, angiopoietin-1, and angiopoietin-2 in a blood, plasma or serum sample obtained from the patient, and

(2) predicting the responsiveness of the patient to the anti-VEGF therapy with reference to the level determined in step (1).

In an aspect, the disclosure provides a diagnostic agent for predicting the responsiveness of a wet AMD patient to anti-VEGF therapy comprising at least one antibody selected from the group consisting of anti-TGF-beta antibody, anti-BMP9 antibody, anti-angiopoietin-1 antibody, and anti-angiopoietin-2 antibody.

The disclosure enables predicting the responsiveness of a wet AMD patient to anti-VEGF therapy. The prediction may be useful for determining whether the patient should be treated with an anti-VEGF agent. When the patient has been treated with an anti-VEGF agent, the prediction may be useful for determining whether the treatment should be continued.

BRIEF DESCRIPTION OF DRAWINGS

Fig. 1 shows the serum TGF-beta levels of the patients in control group, naive group, well response group, and refractory group (** p < 0.01).
Fig. 2 shows the serum BMP9 levels of the patients in control group, naive group, well response group, and refractory group (** p < 0.01).
Fig. 3 shows the serum angiopoietin-1 levels of the patients in control group, naive group, well response group, and refractory group (** p < 0.05).
Fig. 4 shows the serum angiopoietin-2 levels of the patients in control group, naive group, well response group, and refractory group (** p < 0.05).

DETAILED DESCRIPTION

Unless otherwise defined, the terms used herein are read as generally understood by a skilled person in the technical fields such as organic chemistry, medicine, pharmacology, molecular biology, and microbiology. Several terms used herein are defined as described below. The definitions herein take precedence over the general understanding.

When a numerical value is accompanied with the term "about", the value is intended to represent any value within the range of $\pm$ 10% of that value. A numerical range covers all values from the lower limit to the upper limit and includes the values of the both limits. When a numerical range is accompanied with the term "about", the both limits are read as accompanied with the term. For example, "about 20 to 30" is read as "20 $\pm$ 10% to 30 $\pm$ 10%."

The term "anti-VEGF agent" as used herein means an agent that inhibits the function of a VEGF, including, e.g., VEGF inhibitors, VEGF receptor inhibitors, and nucleic acids that inhibit expression of a VEGF. Examples of anti-VEGF agents include ranibizumab, aflibercept, bevacizumab, and pegaptanib.

The term "the responsiveness of a wet AMD patient to anti-VEGF therapy" as used herein means the degree of the remission of the wet AMD patient caused by the anti-VEGF therapy. The degree of the remission may be determined by evaluating hemorrhage or leakage of blood components from CNV, the resulting accumulation of subretinal or intraretinal fluid (retinal edema or detachment of retinal pigment epithelium), or a symptom of wet AMD such as metamorphopsia, reduced visual acuity, central scotoma, defective color vision, or visual field defect. In an embodiment, the responsiveness is determined by evaluating the subretinal or intraretinal fluid or the reduced visual acuity.

The term "a wet AMD patient is responsive to the anti-VEGF therapy" as used herein means that the patient is remitted from wet AMD by the anti-VEGF therapy. For example, the patient is remitted within 12 months, preferably 6 months, more preferably 3 months from the start of the anti-VEGF therapy.

The term "remit" or "remission" as used herein means the cause of wet AMD is partially or completely reduced or removed, the progression of wet AMD is delayed or stopped, and/or a symptom of wet AMD is reduced, alleviated, ameliorated, or removed.

The term "patient" as used herein means an animal suffering from wet AMD. The animal may be a human or non-human animal. Examples of non-human animals include mice, rats, guinea pigs, rabbits, dogs, cats, monkeys, pigs, cattle, and horses, preferably mice, rats, guinea pigs, rabbits, dogs, and monkeys. In an embodiment, the patient is a human.

The term "marker protein" as used herein means a protein that is detected in a blood, plasma, or serum sample obtained from a wet AMD patient and is capable of indicating the responsiveness of the patient to the anti-VEGF therapy according to its level in the sample. The marker protein is at least one selected from TGF-beta, BMP9, angiopoietin-1, and angiopoietin-2 in the disclosure.

TGF-beta is a cytokine which suppresses angiogenesis (Int J Cancer. 2004;108(2):251-7). The representative amino acid sequence of human TGF-beta (SEQ ID NO: 1) is deposited in GenBank (accession number P01137). BMP9 is a cytokine which promotes angiogenesis (PLoS One. 2012;7(1):e30075.; J Cell Sci. 2010;123(Pt 10):1684-92). The representative amino acid sequence of human BMP9 (SEQ ID NO: 2) is deposited in GenBank (accession number Q9UK05). Antagonism between TGF-beta and BMP9 is known.

Angiopoietin-1 is a growth factor which suppresses angiogenesis (Invest Ophthalmol Vis Sci. 2014;55(4):2191-9). The representative amino acid sequence of human angiopoietin-1 (SEQ ID NO: 3) is deposited in GenBank (accession number Q15389). Angiopoietin-2 is a growth factor which promotes angiogenesis (J Clin Invest. 2012;122(6):1991-2005). The representative amino acid sequence of human angiopoietin-2 (SEQ ID NO: 4) is deposited in GenBank (accession number O15123). Antagonism between angiopoietin-1 and angiopoietin-2 is known.

The four marker proteins may include deletion, substitution, or addition at one or several amino acids in their original sequences, for example, the amino acid sequences of SEQ ID NOs: 1 to 4, as long as their functions are maintained. The term "several amino acids" means preferably 2 to 7, more preferably 2 to 5, most preferably 2 to 3 amino acids. The amino acid substitution is preferably conservative substitution between similar amino acids.

The four marker proteins may comprise amino acid sequences having at least about 60%, preferably at least about 70%, more preferably at least about 80%, still more preferably at least about 90%, especially preferably at least about 95%, and most preferably about 97%, about 98%, or about 99% sequence identity with their original sequences, for example, the amino acid sequences of SEQ ID NOs: 1 to 4, as long as their functions are maintained. The sequence identity may be calculated with a program such as BLAST, for example, using BLAST with the parameters set to default values.

A blood, plasma or serum sample obtained from a wet AMD patient is used in the disclosed method. The blood sample may be obtained in a conventional manner, for example from a vein or artery of the patient. The plasma or serum sample may be prepared from the blood sample by any suitable methods known to those skilled in the art. The methods are not limited and may include any clinically acceptable steps, such as addition of an anticoagulant and centrifugation. The blood, plasma, or serum sample may be stored at a low temperature, for example in a freezer, during or after the preparation before the use. The blood, plasma, or serum sample may be diluted before the use as needed.

The levels of each marker protein may be determined with an immunoassay using an antibody capable of specifically binding to the marker protein. Examples of immunoassays include flow cytometry, radioimmunoassay (RIA), enzyme-linked immunosorbent assay (ELISA), western blotting, and immunohistological staining.

The term "antibody" as used herein means an affinity ligand based on an immunoglobulin scaffold. The antibody may be a monoclonal or polyclonal antibody of any origins. The antibody may be, e.g., a mouse, rat, rabbit, goat, or human antibody, or a chimeric antibody comprising sequences from different species, such as a partially humanized antibody, e.g., a partially humanized mouse antibody. The antibody may be produced by a conventional method in which a marker protein or its partial peptide having antigenicity is used as an immunogen. For example, a polyclonal antibody may be produced by immunizing an animal with an antigen. A monoclonal antibody may be produced by hybridoma technology. A commercially available antibody also may be used.

The antibody may be a fragment or derivative thereof that is capable of selectively interacting with the marker protein. Examples of antibody fragments or derivatives include Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL; single chain Fv fragments (scFv), consisting of the two VH and VL domains linked together by a flexible peptide linker; camelid heavy-chain dimers and single variable domains, and single domain scaffolds like e.g., the New Antigen Receptor (NAR) from the nurse shark and minibodies based on a variable heavy domain.

The antibody may be labeled. Those skilled in the art may select a suitable label. Examples of labels include fluorescent dyes or metals (e.g., fluorescein, rhodamine, phycoerythrin, fluorescamine), chromophoric dyes (e.g., rhodopsin), chemiluminescent compounds (e.g., luminal, imidazole), bioluminescent proteins (e.g., luciferin, luciferase), haptens (e.g., biotin), enzymes (e.g., peroxidase, alkaline phosphatase, beta-lactamase), radioisotopes (e.g., $^3$H, $^{14}$C, $^{32}$P, $^{35}$S, $^{125}$I), and particles (e.g., metal particles such as gold), and fluorescent semiconductor nanocrystals (quantum dots). The different types of labels may be conjugated to the antibody using various chemistries known to those skilled in the art, e.g., amine reaction or thiol reaction. Other reactive groups, e.g., aldehydes, carboxylic acids and glutamine may also be used. Alternatively, the antibody may have no label and a labeled secondary antibody that binds to the primary antibody may be employed.

The antibody may be attached to a suitable support to provide an antibody array. Any support may be used irrespective of its shape or material, as long as it allows the attachment of the antibody. Examples of supports include a membrane, e.g. nylon membrane, beads, a glass support, a plastic support, or a metal support.

Once a level of a marker protein is determined, the responsiveness of the wet AMD patient to the anti-VEGF therapy is predicted with reference to the level. For example, the determined level is compared to a level of the marker protein in a blood, plasma, or serum sample from a wet AMD patient who is responsive to the therapy (responder) and/or a level of the marker protein in a blood, plasma, or serum sample from a wet AMD patient who is not responsive to the therapy (non-responder). Alternatively, the determined level may be compared to a predetermined standard level. The standard level may be an average level of the marker protein in blood, plasma, or serum samples from the responders and/or an average level of the marker protein in blood, plasma, or serum samples from the non-responders. Alternatively, the responsiveness may be predicted by comparing the determined level to a distribution of levels of the marker protein in blood, plasma, or serum samples from the responders and/or a distribution of levels of the marker protein in blood, plasma, or serum samples from the non-responders. The determined level is preferably compared on the basis of statistical significance.

The experiments described below proved that the blood levels of TGF-beta and angiopoietin-1 are higher in the responders than in the non-responders. This positive correlation enables predicting the responsiveness of a wet AMD patient to the anti-VEGF therapy. For example, when the level of at least one marker protein selected from the group consisting of TGF-beta and angiopoietin-1 in a wet AMD patient is evaluated to be relatively high, it may be predicted that the patient is more likely to be responsive to the anti-VEGF therapy.

The experiments described below proved that the blood levels of BMP9 and angiopoietin-2 are lower in the responders than in the non-responders. This negative correlation enables predicting the responsiveness of a wet AMD patient to the anti-VEGF therapy. For example, when the level of at least one marker protein selected from the group consisting of BMP9 and angiopoietin-2 in a wet AMD patient is evaluated to be relatively low, it may be predicted that the patient is more likely to be responsive to the anti-VEGF therapy.

Furthermore, the responsiveness of a wet AMD patient to the anti-VEGF therapy may be predicted more accurately with reference to the levels of two, three, or four of the marker proteins. In an embodiment, when the level of TGF-beta is evaluated to be relatively high and the level of BMP9 is evaluated to be relatively low, when the level of TGF-beta is evaluated to be relatively high and the level of angiopoietin-2 is evaluated to be relatively low, when the level of angiopoietin-1 is evaluated to be relatively high and the level of angiopoietin-2 is evaluated to be relatively low, or when the level of angiopoietin-1 is evaluated to be relatively high and the level of BMP9 is evaluated to be relatively low, it may be predicted that the patient is more likely to be responsive to the anti-VEGF therapy. In another embodiment, when the levels of both TGF-beta and angiopoietin-1 are evaluated to be relatively high, or when the levels of both BMP9 and angiopoietin-2 are evaluated to be relatively low, it may be predicted that the patient is more likely to be responsive to the anti-VEGF therapy. In another embodiment, when the levels of both TGF-beta and angiopoietin-1 are evaluated to be relatively high and the level of BMP9 is evaluated to be relatively low, when the levels of both TGF-beta and angiopoietin-1 are evaluated to be relatively high and the level of angiopoietin-2 is evaluated to be relatively low, when the level of TGF-beta is evaluated to be relatively high and the levels of both BMP9 and angiopoietin-2 are evaluated to be relatively low, or when the level of angiopoietin-1 is evaluated to be relatively high and the levels of both BMP9 and angiopoietin-2 are evaluated to be relatively low, it may be predicted that the patient is more likely to be responsive to the anti-VEGF therapy. In another embodiment, when the levels of both TGF-beta and angiopoietin-1 are evaluated to be relatively high and the levels of both BMP9 and angiopoietin-2 are evaluated to be relatively low, it may be predicted that the patient is more likely to be responsive to the anti-VEGF therapy.

Alternatively, the determined level may be compared to a predetermined cutoff value for the blood, serum or plasma level of the marker protein. For example, when the level of at least one marker protein selected from the group consisting of TGF-beta and angiopoietin-1 is not lower than a cutoff value, it may be predicted that the patient is more likely to be responsive to the anti-VEGF therapy. For example, when the level of at least one marker protein selected from the group consisting of BMP9 and angiopoietin-2 is not higher than a cutoff value, it may be predicted that the patient is more likely to be responsive to the anti-VEGF therapy.

The term "cutoff value", also called as "threshold value", means a value that serves as a reference to predict the responsiveness of a patient to the therapy. Preferably, the cutoff value enables high diagnostic sensitivity and high diagnostic specificity. The term sensitivity means true positive rate and the term specificity means true negative rate. For example, the cutoff value may be the blood, serum, or plasma level of a marker protein that is found in the responders at high positive rate and in the non-responders at high negative rate. Plural cutoff values may be given for one marker protein.

A cutoff value may be determined by any known statistic methods. Typically, a cutoff value may be determined by statistically processing the levels of a marker protein in blood, serum, or plasma samples obtained from the responders and the levels of the marker protein in blood, serum, or plasma samples obtained from the non-responders. A cutoff value may be determined by using software for statistical analysis, e.g., SAS Ver. 9.4.

For example, a cutoff value may be determined by receiver operating characteristic analysis (ROC analysis), which is conventionally used for evaluating effectiveness of diagnosing methods. The ROC analysis includes creating an ROC curve by plotting the sensitivity against the false positive fraction (FPF or false positive rate: 1- specificity) at various cutoff settings. When a diagnosing method is not effective at all, the ROC curve is a straight line connecting the lower left corner and the upper right corner. As the effectiveness gets higher, the ROC curve becomes round and gets closer to the upper left corner. When the effectiveness is 100%, the ROC curve passes through the left side and the upper side. In other words, when a diagnosing method enables both of the high sensitivity and high specificity, the ROC curve with an independent variable is close to the upper left corner. Accordingly, one way to determine a suitable cutoff value for a given diagnostic method is to create an ROC curve and identify a cutoff value that provides the point closest to the upper left corner on the ROC curve. An alternative way is creating an ROC curve and assuming an straight line whose area under the curve (AUC) is 0.05, and identifying a cutoff value that provides the point farthest from the straight line on the ROC curve. In other words, the latter way is calculating the values of (sensitivity + specificity -1) at various cutoff settings and identifying the cutoff value at which the Youden Index, the highest value of (sensitivity + specificity -1), is given.

Typically, a cutoff value may be determined by obtaining blood, plasma or serum samples from the responders and the non-responders, determining the levels of each marker protein in the samples, calculating the values of the diagnostic sensitivity and specificity within the range of the determined levels, creating an ROC curve by using commercially available software for statistical analysis, and identifying the level at which the both values of the diagnostic sensitivity and specificity are as close to 100% as possible. Alternatively, a cutoff value may be based on diagnostic efficiency. The diagnostic efficiency means the proportion of the correctly diagnosed cases, including the cases in which the responders are correctly diagnosed as responders and the cases in which the non-responders are correctly diagnosed as non-responders, in all the diagnosed cases. The values of the diagnostic efficiency are calculated within the range of the determined levels of the marker protein and the cutoff value that provides the highest diagnostic efficiency may be identified.

When plural marker proteins are used, one cutoff value that allows to totally evaluate the levels of the plural marker proteins may be employed. For example, such cutoff value may be determined by conducting statistical analysis, e.g., logistic regression analysis, for plural marker proteins.

Once a cutoff value is identified, it may be optionally varied. In other words, a cutoff value may be freely set, depending on conditions, such as the combination of marker proteins, the method for determining and evaluating the level of marker proteins, the clinical purpose, and the desired sensitivity and specificity.

In an aspect, the disclosure provides a diagnostic agent for predicting the responsiveness of a wet AMD patient to anti-VEGF therapy comprising at least one antibody selected from the group consisting of anti-TGF-beta antibody, anti-BMP9 antibody, anti-angiopoietin-1 antibody, and anti-angiopoietin-2 antibody. The diagnostic agent is used for the method described above.

The antibody may be attached to a suitable support to provide an antibody array. Any supports usually used in the field may be used, including a membrane, e.g. nylon membrane, beads, a glass support, a plastic support, or a metal support.

Each component of the diagnostic agent may be provided separately or in a mixture with other component, as a solution in water or a suitable buffer such as phosphate buffered saline (PBS), or as a lyophilized product, in a suitable container.

The diagnostic agent may be provided in a diagnostic kit that further comprises a component or reagent necessary for determining the level of a marker protein. For example, the kit may comprise a labelled second antibody, a chromogenic substrate, a blocking solution, a washing buffer, an ELISA plate, or a blotting membrane.

In an aspect, the disclosure provides a method of predicting the responsiveness of a wet AMD patient to anti-VEGF therapy comprising

(1) obtaining a blood, plasma or serum sample from the patient,
(2) determining the level of at least one marker protein selected from the group consisting of TGF-beta, BMP9, angiopoietin-1, and angiopoietin-2 in the sample, and
(3) predicting the responsiveness of the patient to the anti-VEGF therapy with reference to the level determined in step (1).

In an aspect, the disclosure provides at least one antibody selected from the group consisting of anti-TGF-beta antibody, anti-BMP9 antibody, anti-angiopoietin-1 antibody, and anti-angiopoietin-2 antibody for use in predicting the responsiveness of a wet AMD patient to anti-VEGF therapy.

In an aspect, the disclosure provides use of at least one antibody selected from the group consisting of anti-TGF-beta antibody, anti-BMP9 antibody, anti-angiopoietin-1 antibody, and anti-angiopoietin-2 antibody for manufacturing a diagnostic agent for predicting the responsiveness of a wet AMD patient to anti-VEGF therapy.

In an aspect, the disclosure provides a method of treating wet AMD comprising administering an effective amount of an anti-VEGF agent to a wet AMD patient who has been predicted to be responsive to the anti-VEGF therapy by the diagnostic method of the disclosure. The term "treat" or "treatment" as used herein means a cause of wet AMD is reduced or removed, the progression of wet AMD is delayed or stopped, and/or a symptom of wet AMD is reduced, alleviated, ameliorated, or removed in a wet AMD patient. The effective amount of the anti-VEGF agent is not limited as long as it is sufficient for producing the desired medical effect. For example, from 0.0001 to 20 mg/eye is preferred, from 0.001 to 10 mg/eye is more preferred, from 0.01 to 5 mg/eye is still more preferred, and from 0.1 to 2.5 mg/eye is especially preferred.

In an aspect, the disclosure provides a method of treating wet AMD comprising

(1) obtaining a blood, plasma or serum sample from the patient,
(2) determining the level of at least one marker protein selected from the group consisting of TGF-beta, BMP9, angiopoietin-1, and angiopoietin-2 in the sample, and
(3) predicting the responsiveness of the patient to the anti-VEGF therapy with reference to the level determined in step (2),
(4) administering an effective amount of an anti-VEGF agent to the patient when the patient is predicted to be responsive to the anti-VEGF therapy in step (3).

Examples of anti-VEGF agents include ranibizumab, aflibercept, bevacizumab, and pegaptanib. Suitable dosage and dosage regimen of anti-VEGF agents are well known to those skilled in the art. For example, an anti-VEGF agent may be administered in accordance with directions for administrating the agent, for example those given in a package insert.

In an aspect, the disclosure provides a therapeutic agent for treating wet AMD comprising an anti-VEGF agent which is administered to a wet AMD patient who has been predicted to be responsive to anti-VEGF therapy by the diagnostic method of the disclosure.

In an aspect, the disclosure provides use of an anti-VEGF agent for treating wet AMD in a wet AMD patient who has been predicted to be responsive to anti-VEGF therapy by the diagnostic method of the disclosure.

In an aspect, the disclosure provides use of an anti-VEGF agent for manufacturing a therapeutic agent for treating wet AMD in a wet AMD patient who has been predicted to be responsive to anti-VEGF therapy by the diagnostic method of the disclosure.

In an aspect, the disclosure provides an anti-VEGF agent for use in treating wet AMD in a wet AMD patient who has been predicted to be responsive to anti-VEGF therapy by the diagnostic method of the disclosure.

For example, the disclosure provides the following embodiments;

[1] A method of predicting the responsiveness of a wet AMD patient to anti-VEGF therapy, comprising

(1) determining the level of at least one marker protein selected from the group consisting of TGF-beta, BMP9, angiopoietin-1, and angiopoietin-2 in a blood, plasma or serum sample obtained from the patient, and
(2) predicting the responsiveness of the patient to the anti-VEGF therapy with reference to the level determined in step (1).

[2] The method according to item [1], wherein the level of at least one marker protein is determined with an antibody specific for the marker protein in step (1).

[3] The method according to item [1] or [2], wherein the responsiveness is predicted by comparing the determined level to a cutoff value predetermined for the marker protein in step (2).

[4] The method according to item [3], wherein the patient is predicted to be responsive to the anti-VEGF therapy when the determined level of TGF-beta is higher than a cutoff value for TGF-beta.

[5] The method according to item [3], wherein the patient is predicted to be responsive to the anti-VEGF therapy when the determined level of BMP9 is lower than a cutoff value for BMP9.

[6] The method according to item [3], wherein the patient is predicted to be responsive to the anti-VEGF therapy when the determined level of TGF-beta is higher than a cutoff value for TGF-beta and the determined level of BMP9 is lower than a cutoff value for BMP9.

[7] The method according to item [3], wherein the patient is predicted to be responsive to the anti-VEGF therapy when the determined level of angiopoietin-1 is higher than a cutoff value for angiopoietin-1.

[8] The method according to item [3], wherein the patient is predicted to be responsive to the anti-VEGF therapy when the determined level of angiopoietin-2 is lower than a cutoff value for angiopoietin-2.

[9] The method according to item [3], wherein the patient is predicted to be responsive to the anti-VEGF therapy when the determined level of angiopoietin-1 is higher than a cutoff value for angiopoietin-1 and the determined level of angiopoietin-2 is lower than a cutoff value for angiopoietin-2.

[10] The method according to any one of items [3] to [9], wherein the cutoff value is determined by statistically processing the levels of the marker protein in blood, serum, or plasma samples from the wet AMD patients who are responsive to the anti-VEGF therapy and the levels of the marker protein in blood, serum, or plasma samples from the wet AMD patients who are not responsive to the anti-VEGF therapy.

[11] The method according to any one of items [1] to [10], wherein the patient has not received anti-VEGF therapy for treating wet AMD.

[12] The method according to any one of items [1] to [10], wherein the patient is receiving or has received anti-VEGF therapy for treating wet AMD.

[13] The method according to any one of items [1] to [12], comprising administering an effective amount of an anti-VEGF agent to a wet AMD patient who is predicted to be responsive to the anti-VEGF therapy by the method according to any one of items [1] to [12].

[14] A diagnostic agent for predicting the responsiveness of a wet AMD patient to anti-VEGF therapy, comprising at least one antibody selected from the group consisting of anti-TGF-beta antibody, anti-BMP9 antibody, anti-angiopoietin-1 antibody, and anti-angiopoietin-2 antibody.

The entire contents of the documents cited herein are incorporated herein by reference.

The following example does not restrict or limit the invention and is for illustrative purposes only. The embodiments described above are non-limiting and may be modified without deviating from the scope of the invention as defined by the appended claims.

EXAMPLE 1

The correlation between the serum levels of biomarker candidates and the pathology of wet AMD was investigated for the following patient groups.

Group A: control (n=10)
Group B: naive (n=13)
Group C: well response (n=20)
Group D: refractory (n=14)

The inclusion and exclusion criteria for each group are summarized in the tables below.

[Table 1]

| No. | Inclusion criteria | Group | | | |
|---|---|---|---|---|---|
| | | A | B | C | D |
| 1 | Aged 65 or over and under 85 | Yes | Yes | Yes | Yes |
| 2 | having diagnosed cataract and undergoing a cataract surgery in near future | Yes | No | No | No |
| 3 | having wet AMD in at least one eye | No | Yes | Yes | Yes |
| 4 | having started anti-VEGF therapy (ranibizumab or aflibercept) 12 months before at the latest, having no subretinal fluid and/or intraretinal fluid (including symptoms such as hemorrhage and edema) detected by examination such as OCT, and having better visual acuity than before the start of the therapy | No | No | Yes | No |
| 5 | having started anti-VEGF therapy (ranibizumab or aflibercept) 12 months before at the latest, having received an anti-VEGF agent sequentially in recent three months, having subretinal fluid and/or intraretinal fluid (including symptoms such as hemorrhage and edema) detected by examination such as OCT, and having no better visual acuity than before the start of the therapy | No | No | No | Yes |
| 6 | undergoing anti-VEGF therapy in near future | No | Yes | Yes | Yes |

[Table 2]

| No. | Exclusion criteria | Group | | | |
|---|---|---|---|---|---|
| | | A | B | C | D |
| 1 | having diagnosed wet AMD in at least one eye | Yes | No | No | No |
| 2 | having CNV in at least one eye due to a cause other than wet AMD (e.g., myopic CNV or injury) | Yes | Yes | Yes | Yes |
| 3 | having macular abnormality in at least one eye due to a cause other than wet AMD | Yes | Yes | Yes | Yes |
| 4 | having diagnosed retinal angiomatous proliferation (RAP) in at least one eye | Yes | Yes | Yes | Yes |
| 5 | having received an anti-VEGF agent through any administration route | Yes | Yes | No | No |
| 6 | having received an anti-VEGF agent through an administration route other than intravitreal injection | No | No | Yes | Yes |
| 7 | having undergone vitreous surgery in the eye to be observed | Yes | Yes | Yes | Yes |
| 8 | having undergone photodynamic therapy (PDT) in the eye to be observed | Yes | Yes | Yes | No |
| 9 | having undergone photodynamic therapy (PDT) in recent two years | No | No | No | Yes |

(continued)

| No. | Exclusion criteria | Group | | | |
|---|---|---|---|---|---|
| | | A | B | C | D |
| 10 | having a complicated active inflammatory disease, including ocular infection and ocular allergy, in the eye to be observed, including eyelid | Yes | Yes | Yes | Yes |
| 11 | having undergone surgery selected from the followings in recent 90 days<br>- intraocular surgery in the eye to be observed, excluding vitreous surgery and PDT<br>- any intraocular surgery in the eye not to be observed<br>- any surgery due to a systemic disease | Yes | Yes | Yes | Yes |
| 12 | suffering from a malignant tumor or having suffered from a malignant tumor in recent five years | Yes | Yes | Yes | Yes |
| 13 | having participated in other clinical trial or clinical study in recent 30 days | Yes | Yes | Yes | Yes |
| 14 | being decided to be inadequate to the study by the attending physician | Yes | Yes | Yes | Yes |

Protocol for immune assay

The candidate biomarkers were 16 proteins which were suggested to be involved in angiogenesis. Serum samples were prepared using each 10 ml of blood samples collected from the patients. The levels of the biomarker candidates in the serum samples were measured using Aushon multiplex immunoassay platform (Aushon BioSystems, Billerica, MA). Antibody arrays were prepared by spotting capture antibodies specific to the 16 biomarker candidates on array plates. The samples were incubated for 2 hours on the antibody arrays and the plates were washed four times. Mixtures of biotinylated detection antibodies were added to each well, then the plates were incubated for 30 to 90 min and washed four times. The plates were then incubated with streptavidin-horseradish peroxidase conjugates for 30 min. All the incubation steps were carried out at room temperature with shaking at 200 rpm. The plates were washed again and chemiluminescent substances were added. The images of the plates were immediately acquired using Aushon Cirascan CCD Imaging System and the data were analyzed using Aushon Cirasoft Software.

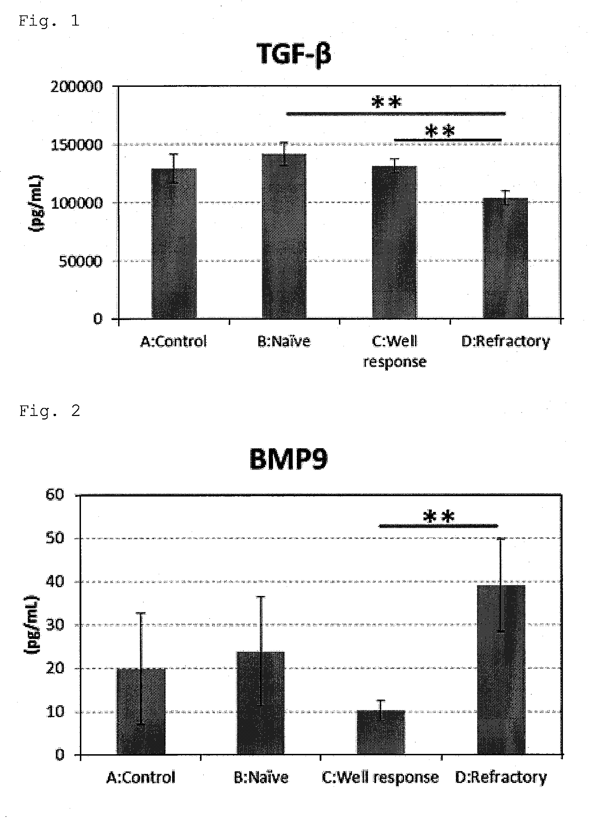

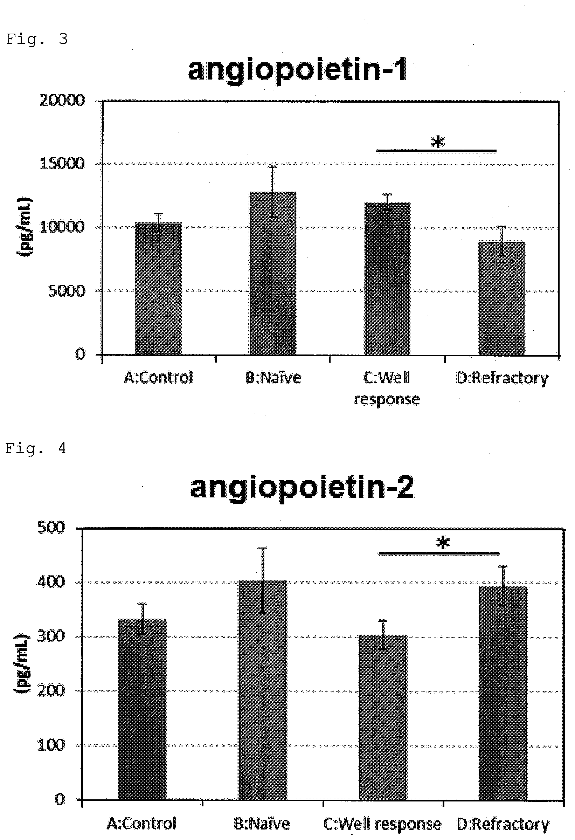

The results are shown in the following tables and Figs. 1-4. The levels of TGF-beta, BMP9, angiopoietin-1, and angiopoietin-2 were significantly different between the well response group and the refractory group. The levels of TGF-beta and angiopoietin-1 were higher and the level of BMP9 and angiopoietin-2 were lower in the well response group.

[Table 3]

| | TGF-beta | | BMP9 | | angiopoietin-1 | | angiopoietin-2 | |
|---|---|---|---|---|---|---|---|---|
| Group | pg/ml | mean | pg/ml | mean | pg/ml | mean | pg/ml | mean |
| A: control | 95940.2 | | 4.5 | | 7411.9 | | 207.0 | |
| | 97144.4 | | 5.0 | | 8182.0 | | 238.8 | |
| | 104633.8 | | 5.3 | | 8464.3 | | 240.7 | |
| | 107036.9 | | 5.4 | | 8685.7 | | 269.0 | |
| | 116713.5 | | 5.5 | | 9462.5 | | 308.0 | |
| | 118855.1 | | 5.7 | | 11194.8 | 10380.1 | 385.8 | |
| | 122668.7 | | 8.6 | | 11506.3 | | 399.3 | |
| | 133271.9 | | 11.2 | | 12105.5 | | 420.6 | |
| | 171512.6 | | 11.4 | | 12163.3 | | 421.6 | |
| | 221510.8 | 128928.8 | 135.3 | 19.8 | 14624.2 | | 428.0 | 331.9 |

[Table 4]

| | TGF-beta | | BMP9 | | angiopoietin-1 | | angiopoietin-2 | |
|---|---|---|---|---|---|---|---|---|
| Group | pg/ml | mean | pg/ml | mean | pg/ml | mean | pg/ml | mean |
| B: naive | 104435.4 | | 4.7 | | 5613.0 | | 208.6 | |
| | 105639.8 | | 5.2 | | 6884.2 | | 223.8 | |
| | 111151.5 | | 5.7 | | 8359.0 | | 281.4 | |
| | 116483.9 | | 6.4 | | 9040.1 | | 290.8 | |
| | 127020.3 | | 7.1 | | 11218.7 | | 316.4 | |
| | 127804.1 | | 8.6 | | 11372.9 | | 332.1 | |
| | 128878.1 | | 9.8 | | 11479.4 | | 367.6 | |
| | 144808.0 | | 12.1 | | 12292.3 | | 367.7 | |
| | 147898.8 | | 14.2 | | 12586.6 | | 393.0 | |
| | 149281.0 | | 14.8 | | 12642.0 | | 423.5 | |
| | 155965.5 | | 15.8 | | 14438.2 | | 473.7 | |
| | 185276.0 | | 32.9 | | 15338.1 | | 511.4 | |
| | 231892.9 | 141272.0 | 171.9 | 23.8 | 34906.5 | 12782.4 | 1056.0 | 403.5 |

[Table 5]

| | TGF-beta | | BMP9 | | angiopoietin-1 | | angiopoietin-2 | |
|---|---|---|---|---|---|---|---|---|
| Group | pg/ml | mean | pg/ml | mean | pg/ml | mean | pg/ml | mean |
| C: well response | 84322.0 | | 4.3 | | 7479.2 | | 111.8 | |
| | 101438.6 | | 4.7 | | 8348.6 | | 147.5 | |
| | 107816.5 | | 5.3 | | 8943.6 | | 197.0 | |
| | 107855.2 | | 5.3 | | 9294.0 | | 201.6 | |
| | 110780.5 | | 5.9 | | 9320.1 | | 211.5 | |
| | 112161.4 | | 6.0 | | 10475.1 | | 233.0 | |
| | 114727.8 | | 6.1 | | 10696.3 | | 248.4 | |
| | 116311.0 | | 6.1 | | 10742.1 | | 255.0 | |
| | 118688.6 | | 6.2 | | 10789.9 | | 286.5 | |
| | 125840.4 | | 6.8 | | 10980.0 | | 291.7 | |
| | 132535.6 | | 6.9 | | 11883.5 | | 293.9 | |
| | 133213.1 | | 7.1 | | 12918.9 | | 307.9 | |
| | 138463.8 | | 7.2 | | 13293.2 | | 329.7 | |
| | 140793.9 | | 7.8 | | 13859.6 | | 332.1 344.4 | |
| | 142329.1 | | 8.0 | | 14005.5 | | | |
| | 154820.2 | | 9.3 | | 14103.1 | | 372.1 | |
| | 155285.0 | | 9.4 | | 14333.7 | | 373.7 | |
| | 157602.9 | | 12.4 | | 15123.5 | | 401.0 | |
| | 170100.8 | | 35.2 | | 16487.9 | | 557.0 | |
| | 200188.3 | 131263.7 | 43.2 | 10.2 | 16992.8 | 12003.5 | 576.3 | 303.6 |

[Table 6]

| | TGF-beta | | BMP9 | | angiopoietin-1 | | angiopoietin-2 | |
|---|---|---|---|---|---|---|---|---|
| Group | pg/ml | mean | pg/ml | mean | pg/ml | mean | pg/ml | mean |
| D: refractory | 66646.4 | | 4.3 | | 3226.1 | | 206.2 | |
| | 74499.7 | | 6.3 | | 3728.2 | | 217.0 | |
| | 80128.6 | | 6.8 | | 5711.6 | | 220.8 | |
| | 84479.9 | | 9.0 | | 6790.6 | | 303.6 | |
| | 87914.6 | | 12.8 | | 7213.8 | | 313.6 | |
| | 97030.8 | | 13.4 | | 7464.5 | | 333.4 | |
| | 107789.4 | | 15.8 | | 7755.9 | | 403.8 | |
| | 108702.8 | | 27.8 | | 8476.2 | | 405.2 | |
| | 111708.6 | | 39.1 | | 8518.6 | | 435.3 | |
| | 116813.9 | | 44.0 | | 9254.7 | | 477.6 | |
| | 119080.9 | | 55.2 | | 9883.6 | | 502.2 | |
| | 126621.5 | | 76.3 | | 11634.5 | | 511.9 | |
| | 133592.8 | | 107.5 | | 17253.2 | | 537.5 | |
| | 135838.8 | 103632.1 | 128.6 | 39.1 | 18327.2 | 8945.6 | 637.4 | 393.3 |

Cut-off values were determined on the basis of the results of Groups C and D using SAS Ver. 9.4 software. Cut-off values were determined for each biomarker so that both of the diagnostic sensitivity and diagnostic specificity were as high as possible. More specifically, each cut-off value was determined so that the distance between the point where both of the sensitivity and specificity are 1 and the point given by the cut off value was the shortest, wherein the distance was calculated as "$\sqrt{((1\text{-sensitivity})^2 + (1\text{-specificity})^2)}$". Using the sample size above, the cut-off values for TGF-beta, BMP9, angiopoietin-1, and angiopoietin-2 were determined as 112161.4 pg/mL, 12.4 pg/mL, 9294.0 pg/mL, and 401.0 pg/mL, respectively.

The cut-off values gave the sensitivities and specificities shown in the table below. More reliable cut-off values could be found if a larger sample size is analyzed.

[Table 7]

| | sensitivity | specificity |
|---|---|---|
| TGF-beta | 75.0% | 64.3% |
| BMP9 | 90.0% | 71.4% |
| angiopoietin-1 | 85.0% | 71.4% |
| angiopoietin-2 | 90.0% | 57.1% |

Furthermore, the multivariate logistic regression analysis in which the four biomarkers are considered simultaneously gave the following model:

```
logit = -3.3987 + (0.000063 × TGF-beta level) - (0.0593 ×
BMP9 level) + (0.000177 × angiopoietin-1 level) - (0.0123 ×
angiopoietin-2 level).
```

The value "logit" means log ((R) / (1 - R)), wherein R is 1 for the well response group and 0 for the refractory group.

When the cut-off value for the logit value was 0.86073, the distance between the point where both of the sensitivity and specificity are 1 and the point given by the cut off value was the shortest. When the cut-off value was used, the sensitivity and specificity were 85.0% and 92.9%, respectively. More reliable cut-off values could be found if a larger sample size is analyzed.

INDUSTRIAL APPLICABILITY

The disclosure enables predicting the responsiveness of a wet AMD patient to anti-VEGF therapy. The prediction may be utilized for selecting therapeutic strategy suitable for the patient. For example, the anti-VEGF therapy may be applied or continued for the patients who are identified as being responsive to the therapy, whereas another therapy may be considered for the patients who are identified as being less responsive to the anti-VEGF therapy.

**Claims**

1. A method of predicting the responsiveness of a wet AMD patient to anti-VEGF therapy, comprising

   (1) determining the level of at least one marker protein selected from the group consisting of TGF-beta, BMP9, angiopoietin-1, and angiopoietin-2 in a blood, plasma or serum sample obtained from the patient, and
   (2) predicting the responsiveness of the patient to the anti-VEGF therapy with reference to the level determined in step (1).

2. The method according to claim 1, wherein the responsiveness is predicted by comparing the determined level to a cutoff value predetermined for the marker protein in step (2).

3. The method according to claim 2, wherein the patient is predicted to be responsive to the anti-VEGF therapy when the determined level of TGF-beta is higher than a cutoff value for TGF-beta.

4. The method according to claim 2, wherein the patient is predicted to be responsive to the anti-VEGF therapy when the determined level of BMP9 is lower than a cutoff value for BMP9.

5. The method according to claim 2, wherein the patient is predicted to be responsive to the anti-VEGF therapy when the determined level of TGF-beta is higher than a cutoff value for TGF-beta and the determined level of BMP9 is lower than a cutoff value for BMP9.

6. The method according to claim 2, wherein the patient is predicted to be responsive to the anti-VEGF therapy when the determined level of angiopoietin-1 is higher than a cutoff value for angiopoietin-1.

7. The method according to claim 2, wherein the patient is predicted to be responsive to the anti-VEGF therapy when the determined level of angiopoietin-2 is lower than a cutoff value for angiopoietin-2.

8. The method according to claim 2, wherein the patient is predicted to be responsive to the anti-VEGF therapy when the determined level of angiopoietin-1 is higher than a cutoff value for angiopoietin-1 and the determined level of angiopoietin-2 is lower than a cutoff value for angiopoietin-2.

9. The method according to any one of claims 1 to 8, wherein the patient has not received the anti-VEGF therapy for treating wet AMD.

10. The method according to any one of claims 1 to 8, wherein the patient is receiving or has received anti-VEGF therapy for treating wet AMD.

11. A diagnostic agent for predicting the responsiveness of a wet AMD patient to anti-VEGF therapy, comprising at least one antibody selected from the group consisting of anti-TGF-beta antibody, anti-BMP9 antibody, anti-angiopoietin-1 antibody, and anti-angiopoietin-2 antibody.

Fig. 1

Fig. 2

Fig. 3

angiopoietin-1

Fig. 4

angiopoietin-2

## INTERNATIONAL SEARCH REPORT

<table>
<tr><td></td><td>International application No.<br>PCT/JP2017/043133</td></tr>
</table>

A. CLASSIFICATION OF SUBJECT MATTER
Int.Cl. G01N33/68(2006.01)i

According to International Patent Classification (IPC) or to both national classification and IPC

B. FIELDS SEARCHED

Minimum documentation searched (classification system followed by classification symbols)
Int.Cl. G01N33/68

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
JSTPlus/JMEDPlus/JST7580(JDreamIII), CAplus/MEDLINE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X/A | JP 2015-516806 A (GENENTECH, INC.) 18 June 2015, claims 1, 8, 13, paragraphs [0092], [0095], [0233] & WO 2013/148288 A1, claims 1, 8, 13, paragraphs [0097], [0102], [0240] & US 2015/0056190 A1 & EP 2830661 A1 & AU 2013240234 A1 & CA 2867588 A1 & CN 104271157 A & KR 10-2014-0142719 A | 1-2, 7, 9-11/3-6, 8 |

☒ Further documents are listed in the continuation of Box C.  ☐ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search<br>05 February 2018 | Date of mailing of the international search report<br>13 February 2018 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2017/043133

C (Continuation). DOCUMENTS CONSIDERED TO BE RELEVANT

| Category* | Citation of document, with indication, where appropriate, of the relevant passages | Relevant to claim No. |
|---|---|---|
| X/A | JP 2015-512612 A (GENENTECH, INC.) 30 April 2015, claims 1, 7, 19, paragraphs [0079], [0082], [0197], table 1 & US 2014/0017233 A1, claims 1, 7, 19, paragraphs [0105], [00108], [0227], table 1 & WO 2013/106765 A1 & EP 2802346 A1 & AU 2013207778 B2 & CA 2862835 A1 & KR 10-2014-0114415 A & CN 104203268 A | 1-2, 9-11/3-8 |
| A | WO 2016/011052 A1 (GENENTECH, INC.) 21 January 2016, claim 1 & JP 2017-523776 A & US 2017/0051360 A1 & EP 3169801 A1 & CN 106460067 A | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62428823 B

**Non-patent literature cited in the description**

- *Graefes Arch Clin Exp Ophthalmol.,* 2014, vol. 252 (4), 647-55
- *Curr Oncol Rep.,* 2014, vol. 16 (2), 365
- *J Clin Oncol,* 2010, vol. 28 (15
- *Int J Cancer.,* 2004, vol. 108 (2), 251-7
- *PLoS One.,* 2012, vol. 7 (1), e30075
- *J Cell Sci.,* 2010, vol. 123, 1684-92
- *Invest Ophthalmol Vis Sci.,* 2014, vol. 55 (4), 2191-9
- *J Clin Invest.,* 2012, vol. 122 (6), 1991-2005